# EUROPEAN PATENT APPLICATION

(11) **EP 4 685 197 A1**
(43) Date of publication of application: **28.01.2026**
(21) Application number: 24774807.2
(22) Date of filing: 13.03.2024
(51) Int. Cl.: C09C 3/00, A61K 8/25, A61Q 1/00, A61Q 1/10, C03C 17/23, C09C 1/28, C09C 3/06, C09D 7/62, C09D 17/00, C09D 201/00

(54) **LUSTROUS MATERIAL**

(30) Priority: 22.03.2023 JP 2023045965
(71) Applicant: NIPPON SHEET GLASS COMPANY, LIMITED, Tokyo 108-6321 (JP)
(72) Inventor: HORIGUCHI, Haruko, Tokyo 108-6321 (JP)
(74) Representative: Cabinet Beau de Loménie
(86) International application number: PCT/JP2024/009891
(87) International publication number: WO 2024/195669

(57) **Abstract**

The present disclosure provides a novel glitter material having a size suitable for use as a lame agent, the glitter material being free of resin pieces and exhibiting favorable color development. The provided glitter material includes a plurality of flaky pieces having a D50 of 100 µm or more in the particle size distribution, and each flaky piece includes a glass flake and a coating film on the glass flake. With respect to 100 flaky pieces, standard deviations σ of a* and b* in a L*a*b* color system of a transmitted color are both 11 or less, the transmitted color being measured using a halogen lamp as a light source.

## Description

### TECHNICAL FIELD

The present invention relates to a glitter material including a plurality of flaky pieces. More specifically, the present invention relates to a glitter material having a size suitable for use as a lame agent in cosmetics, for example.

### BACKGROUND ART

Cosmetics sometimes include glitter materials called lame agents. A lame agent includes pieces for imparting a strong and grainy luster. For the lame agent, usually a laminate of resins having different refractive indices is used. Another example of lame agent is a laminate including a resin substrate on which a metal such as aluminum is coated by vapor deposition. In any case, a lame agent usually includes resin pieces. The lame agent is manufactured, for example, by the method disclosed in Patent Literature 1. A lame agent usually has a size of 100 µm or more, and even hundreds of micrometers to several millimeters.

Another type of glitter material called a pearlescent agent is also known. The pearlescent agent includes pieces for imparting a pearlescent luster by utilizing light interference. Reflected light from the pearlescent agent can impart excellent texture. Usually however, the reflected light from the pearlescent agent cannot impart grainy texture equivalent to that of the lame agent. As the pearlescent agent, inorganic pieces having a light-interference film such as a titanium oxide film formed on the surfaces are usually used. As the substrate for serving as the light-interference film of the pearlescent agent, an inorganic substrate of mica, talc, glass or the like is used. The pearlescent agent usually has a size of approximately several tens of micrometers.

As described above, a lame agent and a pearlescent agent are used as different types of glitter materials.

### CITATION LIST

### Patent Literature

Patent Literature 1: JP 2018-70479,A

### SUMMARY OF INVENTION

### Technical Problem

Marine outflow of discarded plastic products has been a significant issue. In particular, microplastics may have adverse effects on ecosystems. Therefore, it is desirable to eliminate resin from a glitter material used as a lame agent. As a glitter material that is free of resin pieces and that can be used as a lame agent, a glitter material including large-particle-sized mica coated with a titanium oxide film is known. However, the color of such a glitter material is slightly dull and insufficient in chroma.

Therefore, the present invention aims to provide a novel glitter material having a size suitable for use as a lame agent, being free of resin pieces, and exhibiting favorable color development.

### Solution to Problem

The present inventors analyzed the factors to cause the color dullness and have found that uniformity of color exhibited by individual pieces can reduce color dullness and improve chroma, thereby completing the present invention. Glass flakes have been known for the usefulness as a substrate in a pearlescent agent for which transparency is emphasized, and the flakes also has high potential as a substrate for a glitter material used as a lame agent.

That is, a first aspect of the present invention provides a glitter material including a plurality of flaky pieces having a D50 of 100 µm or more, wherein
the flaky pieces each include a glass flake and a coating film on the glass flake, and
with respect to 100 flaky pieces, standard deviations σ of a* and b* in a L*a*b* color system of a transmitted color are both 11 or less, the transmitted color being measured using a halogen lamp as a light source.

A second aspect of the present invention provides a glitter material including a plurality of flaky pieces having a D50 of 100 µm or more, wherein
the flaky pieces each includes a glass flake and a coating film on the glass flake, and
the glitter material has a D10 of 65 µm or more.

The D10 and the D50 are defined as particle diameters at 10% and 50%, respectively, of a cumulative volume from a smaller particle diameter in a particle size distribution measured by a laser diffraction/scattering method.

### Advantageous Effects of Invention

According to the present invention, it is possible to obtain a glitter material being free of resin pieces and suitable for use as a lame agent.

### BRIEF DESCRIPTION OF DRAWINGS

FIG. 1 is a schematic view illustrating an example of a device for manufacturing glass flakes.
FIG. 2 is a schematic view illustrating another example of a device for manufacturing glass flakes.

### DESCRIPTION OF EMBODIMENTS

Hereinafter, the present invention will be described in detail, though the following description is not intended to limit the present invention to specific embodiments. In the following description, "D10" and "D50" are as described above, and "D90" is defined similarly as a particle diameter at 90% of a cumulative volume from a smaller particle diameter in a particle size distribution measured by a laser diffraction/scattering method. "Main surface" refers to two surfaces of a piece, each having a relatively large area and a distance between the main surfaces corresponds to the thickness of the piece. "End face" refers to a surface extending in a direction of the thickness and connecting the peripheries of the two main surfaces. "Main component" refers to a component with the highest content in terms of mass. "Metal" is used in accordance with common practice in the field to include semiconductors such as silicon. The same applies to "metal" in a "metal compound," a "metal oxide" or the like.

A hue of a color is indicated by a hue angle h of a L*C*h of color system, specifically, "reddish" refers to 0° or more and less than 45° and 315° or more and less than 360°, "yellowish" refers to 45° or more and less than 135°, "greenish" refers to 135° or more and less than 225°, and "bluish" refers to 225° or more and less than 315°. Here, h is in a relation with a* and b* in a L*a*b* color system such that h = tan⁻¹(b*/a*). The chroma can be expressed using c*, which corresponds to the square root of the sum of the squares of a* and b*.

The glitter material according to the present invention include a plurality of flaky pieces having a D50 of 100 µm or more. The flaky pieces each include a glass flake and a coating film on the glass flake. The coating film may coat the glass flake.

The flaky piece having a D50 of 100 µm or more has a size suitable for use as a lame agent. The D50 may be 120 µm or more, 130 µm or more, 135 µm or more, 140 µm or more, or in some cases, may be 150 µm or more. The upper limit of the D50 is not particularly limited, but is, for example, 3 mm or less, or further, 500 µm or less.

The flaky piece has a D10 of preferably 65 µm or more. In an aggregate of pieces having such a high D10, the ratio of small-particle-sized pieces is limited. Compared to a large-particle-sized piece, a small-particle-sized piece has a higher ratio of end face to the whole surface of the piece. Therefore, by limiting the ratio of the small-particle-sized pieces, the ratio of end face to the whole surface of the glitter material can be reduced. The D10 may be 68 µm or more, 70 µm or more, 72 µm or more, or in some cases 75 µm or more. The upper limit of the D10 is not particularly limited, and it is, for example, 150 µm or less, 120 µm or less, or in some cases 90 µm or less.

It should be noted that the D10, the D50, and the subsequently described D90 of the flaky piece can be adjusted by controlling the particle size distribution of the glass flakes. Usually, the thickness of a coating film is on the order of submicrons, which is significantly smaller than the average particle size (D50 ≥ 100 µm) of the glass flake. In such a case, the particle size distribution of the flaky pieces can be considered substantially the same as the particle size distribution of the glass flake. Even if the influence of an extremely thin coating film is taken into consideration, the measured values of the D10, the D50 or the like of the flaky pieces obtained by forming a coating film on the glass flakes do not exceed the corresponding measured values before formation of the coating film.

The D90 of the flaky piece is not particularly limited, but it may be 200 µm or more. The D90 may be 230 µm or more, and further 250 µm or more. However, a piece formed of a glass flake as a substrate and having an excessively large particle size may be significantly curved. From this viewpoint, the D90 may be 7 mm or less, 5 mm or less, 1 mm or less, 700 µm or less, 500 µm or less, 450 µm or less, or in some cases 400 µm or less.

A value of a ratio of the D90 to the D10, namely, D90/D10 may be used as an indicator for narrowness of a particle size distribution. In the case of a small-particle-sized glitter material such as a pearlescent agent, the grainy texture in the exhibited color can be improved by setting the upper limit of the D90/D10 to a specified value (for example, 3). However, as to the glitter material of the present invention, there is little need to exclude large-particle-sized pieces, and thus, it is not required to limit the D90/D10 to 3 or less. The D90/D10 may be, for example, more than 3 and 6 or less, or even more than 3 and 5 or less. The lower limit of the D90/D10 may be 3.1 or more, or in some cases, 3.2 or more.

There is no particular limitation on the thickness of the flaky piece, but the thickness may be 0.5 µm or more, 1.0 µm or more, 7.0 µm or less, or 5.0 µm or less.

A glass flake is a piece of glass also known as a scale-shaped glass or the like. The glass flake is amorphous, and its surfaces, particularly its main surfaces, exhibit superior smoothness compared to a crystalline substrate. A crystalline inorganic piece of mica, talc or the like tends to have a step on its surface. When the size of the crystalline inorganic piece increases, a step appears, especially on the main surface, due to inevitable cleavage. Use of the glass flake as a substrate is significant for eliminating such a step on the surface, especially on the main surface.

As to resin pieces manufactured by cutting a resin matrix, the cross sections, namely the end faces, become flat. In contrast, flaky inorganic pieces are usually manufactured by crushing or pulverizing an inorganic matrix. Therefore, a flaky inorganic piece has an irregular shape, and few parts on the end faces can be considered planar. The limitation on the ratio of small-particle-sized pieces in flaky inorganic pieces is significant in reducing the ratio of irregularly crushed surfaces to the total surface area of the pieces.

The end face and the step on the main surface not only cause light scattering on their own, but, on a coating film formed on the step or on the end face, light enters at an incident angle different from the incident angle of light entering on a coating film on a smooth main surface. Especially in a case where the coating film is a film that exhibits color due to light interference, a mixture of multiple colors tends to be observed when the angle of light incidence, namely, the optical path length in the coating film, is not constant. Light scattering and the exhibited multiple colors reduce the chroma of the color exhibited by the glitter material, resulting in a dull appearance as a whole.

Glass flakes are well known. There is no particular limitation on the composition of glass flakes. Glass flakes may be formed, for example, of various multi-component glasses containing silica as a main component.

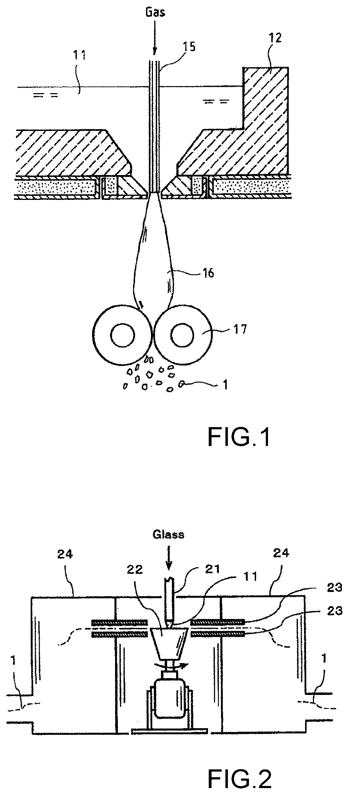

A method for manufacturing glass flakes is described with reference to FIG. 1 and FIG. 2. FIG. 1 shows an example of a device for performing a so-called blowing process. In the device shown in FIG. 1, a glass material 11 melted in a refractory tank furnace 12 is inflated into a balloon shape by a gas fed through a blow nozzle 15, thereby forming a hollow glass film 16. The hollow glass film 16 is then pulverized with a pair of pressure rolls 17, whereby glass flakes 1 are obtained. FIG. 2 shows an example of a device for performing a so-called rotary process. In the device shown in FIG. 2, a molten glass material 11 is poured into a rotating cup 22 through a nozzle 21 and radially discharged from the upper edge of the cup 22 due to the centrifugal force generated by the rotation of the cup 22. The discharged material 11 is sucked by an air flow through annular plates 23, 23 arranged above and below, and introduced into an annular cyclone-type collector 24. In passing through the annular plates 23, 23, the glass is cooled and solidified into a thin film, and is further crushed into fine pieces, whereby a glass flake 1 is obtained.

The particle size distribution of the glass flake can be adjusted, for example, by controlling the crushing or pulverizing process and, classification may be performed as required. An example of the classification is sieve classification by use of a sieve having an appropriate opening size. Sieve classification may be performed using a single sieve or a plurality of sieves. An example of the latter case is sieve classification using two sieves, a first sieve having a relatively small opening and a second sieve having a relatively large opening. Methods for adjusting the particle size distribution is not limited to the examples, but adjustment may be performed using known dry or wet classification device.

The coating film may contain a metal compound. The metal compound may be a nitride, a carbide or the like, and preferably an oxide. Examples of metal oxides include titanium oxide, iron oxide, cerium oxide, silicon oxide, aluminum oxide, zinc oxide, zirconium oxide, niobium oxide, and tin oxide. The coating film preferably includes a metal oxide, particularly titanium oxide.

The coating film may be formed of a single layer or a plurality of layers. Regardless of whether the film is formed of a single layer or a plurality of layers, the present invention is particularly suitable for application to a glitter material having a film that exhibits, as a coating film, a color through light interference. The coating film that exhibits interference colors usually has at least one thin film of a metal compound, particularly a metal oxide.

The coating film that exhibits interference colors may be formed of a plurality of layers including a high-refractive-index layer with a relatively high refractive index and a low-refractive-index layer with a relatively low refractive index. The materials constituting these layers are not particularly limited, but the material constituting the high refractive index layer is, for example, at least one selected from the group consisting of titanium oxide, zirconium oxide, iron oxide, cerium oxide, zinc oxide, niobium oxide, and tin oxide, and the material constituting the low refractive index layer is, for example, at least one selected from the group consisting of silicon oxide and aluminum oxide.

The thickness of the coating film may be appropriately adjusted in accordance with the composition and materials of the coating film so that the desired color is achieved. The thickness of the coating film is usually less than 1 µm, for example, 20 nm or more and 300 nm or less, and particularly 50 nm or more and 200 nm or less.

Titanium oxide has a high refractive index and is suitable for forming a film with excellent color development. Titanium oxide has three types of crystal structures, namely anatase-type, brookite-type, and rutile-type structures, and the anatase-type titanium oxide and the rutile-type titanium oxide are mass-produced industrially. The rutile-type structure is preferred among the crystal structures of titanium oxide. The rutile-type titanium oxide has low photocatalytic activity, making it less likely to affect matrix materials such as paints containing a glitter pigment, and the rutile-type titanium oxide has the highest refractive index.

Formation of the rutile-type titanium oxide film on the glass flakes can be performed according to a method disclosed, for example, in JP 2001-31421,A or JP 2003-12962,A. In the method disclosed in these patent publications, rutile-type titanium oxide is precipitated on glass flakes in a solution containing a titanium compound such as titanium tetrachloride, and thus a coating film is formed on each glass flake. Precipitation of the rutile-type titanium oxide on the glass flakes can be caused by adding an alkaline compound or an alkaline solution to the titanium compound-containing solution having a temperature of 55 to 85°C and a pH of 1.3 or less. A tin treatment, namely, preliminarily attaching tin or a tin compound to the glass flakes facilitates precipitation of the rutile-type titanium oxide. This method can also be used as a technique for forming a rutile-type titanium oxide film on glass flakes coated previously with fine gold particles. With the use of this method, a rutile-type titanium oxide film can be formed without the need for heating for crystal transformation.

A titanium oxide film exhibits a color corresponding to its thickness, usually exhibiting a yellowish tone at approximately 100 nm thickness, a reddish tone at approximately 130 nm thickness, a bluish tone at approximately 160 nm thickness, and a greenish tone at approximately 175 nm thickness.

According to another technique known in the field of pearlescent agents, fine gold particles are attached to a surface of a titanium oxide film so as to utilize surface plasmon resonance imparted by the fine gold particles, thereby improving the chroma that is often insufficient. However, in one aspect of the present invention, the glitter material achieves an advantage of exhibiting colors of high chroma without fine gold particles attached to the surface of the coating film.

In one aspect of the present invention, the uniformity of color exhibited by the flaky pieces constituting the glitter material is sufficiently high. Specifically, with respect to 100 flaky pieces, standard deviations σ of a* and b* in a L*a*b* color system of a transmitted color can both be decreased to 11 or less. The standard deviation σ can be decreased to 10.5 or less, 10 or less, or in some cases 9 or less, and further to 8.5 or less. The lower limit of the standard deviation σ is not intended to limit the range of σ, but for example, σ of a* and b* may each be 3 or more, 4 or more, or further 5 or more, and the larger of the two σ values for a* and b* may be 7 or more. The values of a* and b* for each flaky piece can be measured using a halogen lamp as the light source and a polarization microscope. The details of this measurement method are described in

### EXAMPLES.

Uniformity of the hue of each color allows for unpredictable improvement in the chroma of the color. The chroma, which is specifically indicated by c*, may reach 30 or higher, 31 or higher, or in some cases 32 or higher. The upper limit of the chroma c* is, for example, 37, although this is not intended to be a limitation. The chroma c* of the glitter material can be measured by preparing a specified test panel. The details of this measurement method are also described in EXAMPLES.

The glitter material according to the present invention can be blended in use for not only cosmetics but various compositions. From another aspect, the present invention provides a glitter-material-containing composition including the glitter material according to the present invention. Examples of glitter-material-containing compositions include at least one selected from paints, inks, cosmetics, and resin compositions. Examples of resin compositions include molded products of artificial marble.

Furthermore, the present invention provides, from another aspect, a glitter-material-containing coated body including a substrate and a coating, where the coating formed on the substrate contains the glitter material according to the present invention. The glitter-material-containing coated body may be a coated paper. The substrate is paper in this case, but the substrate is not limited to paper and may be metal, resin, ceramics, or the like. The coating may be formed of the glitter-material-containing composition of the present invention or may be formed by applying the glitter-material-containing composition according to the present invention onto the substrate.

Since specific examples of the glitter-material-containing composition and the glitter-material-containing coated body are well known, explanations thereof is omitted here except for the following description related to cosmetics.

Examples of cosmetics include facial cosmetics, makeup cosmetics, and hair cosmetics. In particular, the glitter material of the present embodiment is preferably used in makeup cosmetics such as eye shadows, nail enamels, eyeliners, mascaras, lipsticks, and face powders. The form of cosmetics is not particularly limited, but the examples include powders, cakes, pencils, sticks, ointments, liquids, emulsions, and creams.

### EXAMPLES

The present invention will now be described in more detail with reference to Examples. First, specific evaluation methods will be described.

### (D10, D50, D90)

The particle size distribution of a powder of flaky pieces prepared by forming a coating film on a surface of a glass flake was measured using a laser diffraction/scattering method. A Microtrac MT3300EXII manufactured by Microtrac Bel Corp. was used for the measurement. The particle sizes corresponding to 10%, 50%, and 90% of the cumulative volume from the smaller particle size side were defined as D10, D50, and D90, respectively.

### (Color evaluation of flaky piece)

A powder of flaky pieces was observed and photographed using a polarization microscope. A sample to be observed was prepared by placing a small amount of powder on a microscope slide and sandwiching the sample between the microscope slide and a cover glass so that the individual pieces overlapped as little as possible. The polarization microscope used was CX31-P manufactured by Olympus Corporation. A 6V 30W halogen lamp was used as the light source. The color temperature of this halogen lamp is 3200 K. It should be noted that the polarization microscope was used without inserting either an upper Nicol prism or a lower Nicol prism. Photographs were taken in the state where approximately 100 to 300 flaky pieces were present in the field of view. In observations using the polarization microscope, the transmitted color of the sample is observed.

In the image data obtained by photographing, the color tone of the transmitted light was evaluated for 100 pieces in descending order of size, though evaluation was omitted for pieces that were out of focus due to a large inclination or due to curved main surfaces. The measurement was performed while overlapped pieces were excluded from the measurement. In a case where transmitted lights of different color tones were observed on a single piece, specifically in a case where interference fringes were observed, the color tone was evaluated based on the color tone with the larger area proportion. To evaluate the color tone, image data was input into https://www.color-site.com/image_pickers to obtain RGB data, which was then converted into L*, a*, b*. The conversion to L*, a*, b* was performed at https://www.webtoolss.com/colorcode2.html. While use of these websites is not mandatory, the websites are useful for quantifying color tones.

### (Color evaluation of coated panel)

A coated panel was prepared, the panel had a coating containing a powder of flaky pieces to be evaluated. The coating composition was prepared by mixing 10 mass% of the powder with a transparent acrylic resin (NIPPE ACRYL "Auto Clear Super" manufactured by Nippon Paint Co., Ltd.). The coating composition was applied to a white/black opacity chart, allowed to dry to form a coating. Though the composition was applied to achieve a thickness of 9 mil (approximately 228.6 µm), the thickness of the dried coating was 70 to 80 µm. For a black-coated surface, color measurement was performed using a colorimeter CR-400 (manufactured by Konica Minolta, Inc.) to measure L*, a*, b* in the vertical reflection. The light source used was a D65 light source. The square root of the sum of the squares of a* and b* was calculated to obtain c* (chroma).

### (Visual evaluation of coated plate)

An LED light source was positioned so that light entered the coating of the thus prepared coated plate at an incident angle of 45°. The reflected light was visually observed from the direction of the emission angle of 45°. In the case where the proportion of the glitter material exhibiting interference colors other than a single interference color was felt to be less than about 30%, it was judged as "single color". And in the case where the proportion of the glitter material exhibiting interference colors other than a single interference color was felt to be about 30% or more, it was judged as "mixed color."

### [Production of glass flake]

Glass flakes were prepared to have a specified particle size distribution, specifically such that the D10, the D50, and the D90 were the values shown in Table 1, and then, the glass flakes were further classified. The classification was performed by the aforementioned sieving method using two sieves. The thickness of the glass flakes was adjusted to be 1.3 µm in each case.

### [Titanium oxide coating treatment]

The glass flakes with an adjusted particle size distribution were subjected to the tin treatment according to the method described in the aforementioned publication to precipitate rutile-type titanium oxide. After the tin treatment, the glass flakes were subjected to a titanium oxide coating treatment. The titanium oxide coating treatment was performed by dispersing a powder of glass flakes in an aqueous acidic solution adjusted to pH 1.0 with hydrochloric acid, and then while stirring the solution so as to maintain the pH at 1.0, adding dropwise an aqueous titanium tetrachloride solution and an aqueous NaOH solution, thereby precipitating titanium oxide on the surface of the glass flakes. The dropwise addition was stopped at the time the interference color caused by the precipitated titanium oxide visually matched the specified color system (reddish or yellowish). The powder was then separated from the aqueous solution, washed, and dried at 180°C. Thereby, a powder of flaky pieces formed of glass flakes coated with a rutile-type titanium oxide film was obtained.

In this way, the glitter materials of Examples 1 to 6, Comparative Example 1, and Reference Example were obtained. In Reference Example, glass flakes of a size equivalent to that of a pearlescent agent were used. For Comparative Example 2, 7000G manufactured by CQV Co., Ltd. was employed. This is a glitter material including mica coated with titanium oxide, and its size is approximately 60 to 700 µm.

The particle size distributions and evaluation results for the respective glitter materials are shown in Table 1. As to the coatings of Examples 1 to 6, a single color of chroma of c* of 32 or higher, and in some cases 33 or higher, was observed. In contrast, Comparative Example 1 and Comparative Example 2 were inferior in color vividness, because the glitter material in Comparative Example 1 included a number of pieces having a small diameter, and Comparative Example 2 used a crystalline substrate. Vivid color emission was not obtained even in Reference Example where the pearlescent agent was used.

**[Table 1]**

| | Coating film interference color | Particle size distribution | | | | Color distribution |
|---|---|---|---|---|---|---|
| | | D10 | D50 | D90 | D90/D10 | σ(a*) |
| Example 1 | Red | 86.7 | 204.9 | 377.5 | 4.35 | 6.98 |
| Example 2 | Red | 105.3 | 207.8 | 352.2 | 3.34 | 8.16 |
| Example 3 | Red | 75.13 | 150.7 | 251.6 | 3.34 | 8.17 |
| Comparative Example 1 | Red | 61.35 | 140.7 | 246.5 | 4.02 | 11.85 |
| Reference Example | Red | 22.64 | 42.68 | 75.15 | 3.32 | 13.20 |
| Example 4 | Yellow | 103.2 | 212.7 | 332.6 | 3.22 | 9.49 |
| Example 5 | Yellow | 94.9 | 203.3 | 360.2 | 3.80 | 9.90 |
| Example 6 | Yellow | 96.08 | 206.6 | 369.6 | 3.84 | 9.73 |
| Comparative Example 2 | Yellow | - | - | - | - | 15.99 |

**Table 1 (Continued)**

| | Color distribution | Coating observation | | | | |
|---|---|---|---|---|---|---|
| | σ(b*) | L* | a | b* | c* | Observed color |
| Example 1 | 6.51 | 42.46 | 33.19 | -11.99 | 35.3 | Single |
| Example 2 | 6.85 | 45.59 | 32.02 | -10.27 | 33.6 | Single |
| Example 3 | 6.56 | 52.82 | 33.24 | -4.16 | 33.5 | Single |
| Comparative Example 1 | 9.35 | 45.75 | 29.19 | -4.65 | 29.6 | Mixed |
| Reference Example | 7.74 | 45.52 | 29.39 | -3.99 | 29.7 | Mixed |
| Example 4 | 6.08 | 63.24 | -1.97 | 36.49 | 36.5 | Single |
| Example 5 | 5.78 | 65.13 | -2.91 | 32.5 | 32.6 | Single |
| Example 6 | 5.80 | 62.43 | -1.22 | 36.27 | 36.3 | Single |
| Comparative Example 2 | 9.74 | 52.72 | 0.62 | 10.93 | 10.9 | Mixed |

### [Production of eye shadow]

Extender pigments and spherical powders of Category A shown in Table 2 were placed in a disposable cup and mixed with a spatula. The mixture was then introduced into a mixer and mixed for one minute. Further, coloring pigments and glitter materials were introduced, and the mixture was further mixed in the mixer for 30 seconds. Subsequently, raw materials of Category B were introduced into the mixer to be mixed for an additional one minute. The thus obtained power was taken out from the mixer and an appropriate amount of the powder was placed in a mold to be pressed.

**[Table 2]**

| (mass part) | | | | | |
|---|---|---|---|---|---|
| Category | Classification | Product name (raw material) | Manufacturer | Formulation 1 | Formulation 2 |
| A | Extender pigment | JA-46R (talc) | Asada Milling Co., Ltd. | 8.5 | 8.5 |
| | | GMS-4C (sericite) | KINSEI MATEC CO.,LTD. | 1.6 | 1.6 |
| | | NK-10G (synthetic mica) | Nihon Koken Kogyo Co., Ltd. | 1.6 | 1.6 |
| | Color pigment | IRS30S03X | Nippon Sheet Glass Co., Ltd. | 0.2 | 0.2 |
| | Spherical powder | IWS22S13 | Nippon Sheet Glass Co., Ltd. | 0.8 | 0.8 |
| | Glitter material | Example 2 | - | 5.2 | |
| | | Reference Example | - | | 5.2 |
| B | Oil component | NOMUCOAT (a-olefin oligomer) | Nisshin Oillio Group, Ltd. | 0.20 | 0.20 |
| | | P-150 (vaseline) | NIKKO RICA CORPORATION | 1.20 | 1.20 |
| | | KF-96-6 (silicone oil) | Shin-Etsu Silicone | 0.40 | 0.40 |
| | Surfactant | COSMOL 182V (Sorbitan sesquiisostearate) | Nisshin Oillio Group, Ltd. | 0.20 | 0.20 |
| | Antioxidant | E-Mix D | Eisai Food & Chemical Co., Ltd | 0.01 | 0.01 |
| | Preservative | Ethyl paraben | Tokyo Chemical Industry | 0.07 | 0.07 |
| Total | | | | 20 | 20 |

Formulations 1 and 2 were applied to the back of the hand and visually evaluated. Formulation 1 resulted in a design with a noticeable glittery appearance. In contrast, the glitter material of Formulation 2 gave an impression of having a relatively small number of particles, and failed to impart a noticeable glittery appearance.

## Claims

1. A glitter material comprising a plurality of flaky pieces having a D50 of 100 µm or more, wherein
the flaky pieces each comprise a glass flake and a coating film on the glass flake, and
with respect to 100 flaky pieces, standard deviations σ of a* and b* in a L*a*b* color system of a transmitted color are both 11 or less, the transmitted color being measured using a halogen lamp as a light source,
where the D50 is defined as a particle diameter at 50% of a cumulative volume from a smaller particle diameter in a particle size distribution measured by a laser diffraction/scattering method.

2. The glitter material according to claim 1, having a D10 of 65 µm or more, where the D10 is defined as a particle diameter at 10% of the cumulative volume from the smaller particle diameter in the particle size distribution.

3. A glitter material comprising a plurality of flaky pieces having a D50 of 100 µm or more, wherein
the flaky pieces each comprise a glass flake and a coating film on the glass flake, and
the glitter material has a D10 of 65 µm or more,
where the D10 and the D50 are defined as particle diameters at 10% and 50%, respectively, of a cumulative volume from a smaller particle diameter in a particle size distribution measured by a laser diffraction/scattering method.

4. The glitter material according to claim 1 or 3, having a D90 of 1 mm or less,
where the D90 is defined as a particle diameter at 90% of the cumulative volume from the smaller particle diameter in the particle size distribution.

5. The glitter material according to claim 1 or 3, wherein
a ratio of the D90 to the D10 is more than 3 and 5 or less.

6. The glitter material according to claim 1 or 3, wherein
the coating film comprises a metal oxide.

7. The glitter material according to claim 6, wherein
the metal oxide is titanium oxide.

8. A glitter material-containing composition comprising the glitter material according to claim 1 or 3.

9. A glitter material-containing coated body comprising a substrate and a coating formed on the substrate, the coating comprising the glitter material according to claim 1 or 3.
